# EUROPEAN PATENT APPLICATION

(11) **EP 1 686 179 A1**
(43) Date of publication of application: **02.08.2006**
(21) Application number: 05075204.7
(22) Date of filing: 26.01.2005
(51) Int. Cl.: C12N 15/85, A01K 61/00

(54) **Means and methods for improving the development and maturation of eggs and/or sperm in fish using hormones produced by transplanted cells**

(71) Applicant: Universiteit Leiden, 2312 AV Leiden (NL)
(72) Inventor: Spaink, Herman Pieter, 2341 SG Oegstgeest (NL); van den Thillart, Guido Everad Elisabeth Johannes, 2353 EA Leiderdorp (NL); Schnabel Peraza, Denhi, 2313 MJ Leiden (NL)
(74) Representative: Winckels, Johannes Hubertus F.

(57) **Abstract**

The invention is concerned with means and methods for improving the development and/or maturation of eggs and/or sperm in fish using hormone administration comprising providing said fish with cells producing said hormone. Preferred hormones are fertility hormones such as luteinizing hormone (LH), follicle stimulating hormone (FSH)or chorionic gonadotropin (CG) or a functional part, derivative and/or analogue thereof.

SEQUENCE LISTING

<110>  Universiteit Leiden

<120>  Means and methods for improving the development and maturation of
       eggs and/or sperm in fish using hormones produced by
       transplanted cells

<130>  P71812EP00

<140>  EP 05075204.7
<141>  2005-01-27

<160>  15

<170>  PatentIn version 3.3

<210>  1
<211>  958
<212>  DNA
<213>  Artificial

<220>
<223>  LHbeta sequence

<400>  1
atatataaat ctggacacgc agagacactt acaacagcct gctgagcaac cgcaacgcct      60

gtcaagatgt tattggctgg aaatggtgtc ttctttctct tctctttgtt tttcctgctg     120

gcggctgctc agagcttggt ttttccacgc tgtgagctag taaatgagac ggtatcggtg     180

gaaaaagagg gctgtccaaa atgcctggtg tttcagacca ccatctgcag cggccactgc     240

gtaacaaggg atcccgttta caagagcccg ttttccaccg tccaccagac agtgtgcatg     300

taccgggacg tccgctatga gaccattaac ctgcccgact gttccgccgg cgtggacccg     360

cagatcacat acccggtggc gctgagctgc gactgcagtc tgtgcaccat aaacacttcc     420

gactgcacca tccagagcct gcagcccgac ttctgcatgt cccagagaga ggatttcccc     480

gcatactaga cctcgggcaa ctcacgtcaa cctacgcaca tagtcgagct cagcattatt     540

agccctcctg tatgtttttt ccattaatat atatactttc aagacactag tattcagctt     600

aaagtgacat ttaaagacta aactaggtta attaggggga aaagtagagt aagtcattgt     660

ataatagtgg tttgttctgg agacaatcca aaactaatat tgcttaaggg ggctaataaa     720

attgacctta aaatgaattt aaataattta aaaactgcat ttattctagt cgaaataaaa     780

gaaataagac tttctttaga agaaaaaaca ttataggaaa tactgcaaaa aaattcctga     840

atctgttcaa catcattcgg gaaatcaaag gagggctaat aactgtgact tcagctgtac     900

atcaataaag aggctggttc ttaaattcaa aaaaaaaaaa aaaaaaaaaa aaaaaaaa       958

<210> 2
<211> 1038
<212> DNA
<213> Artificial

<220>
<223> FSHbeta sequence

<400> 2
ggtctccacg aaactcccgc agatgaggat gcgtgtgctt gttctggcgc tgctgttgcc 60

ggtgttaatg agcgcagaat cagaatgcag gtgcagctgt cgactcacca acatctccat 120

cactgtggag agcgaagaat gtgggagctg cgtcacaatc gacaccacag cctgtgcagg 180

actatgctgg acaatggatc gagtttaccc tagttccatg gcacagcaca cccagaaggt 240

ctgtaacttc aagaacttga tgtacaagag ctacgagttt aaaggctgtc ctgcaggggt 300

tgattcagtc ttcgtgtacc ccgtggctct gagctgtgag tgcaaccagg ttaactcaga 360

cacaacagac tggggagcta tcagcccgca gaccaccagc tgcagcatac actagagcac 420

tgtatcatga ccttaacaac atgtacgttg cagaatcaaa ttaagtaagg agtacaatta 480

gaccatttaa ggatatcaat tatttacaaa acctttagtt tttcatgcat cccacacaca 540

tggtaatttg gttacttgaa ttaatctgtt gtgttaattc tatagttggt actatggtaa 600

ctagagtact agagtatcca atgctatact agttttaatt acagttaatt atagaaaagt 660

atgctacagt atttattaca gtttttctgt tttcaatatt tagtactaca gtatgctagt 720

gcattcatta acaataagct gtaaatacta taataaatac aggttaatac actttactat 780

agtatgcttg atcaacacta ttatttaatg tgagttacta tagtactttt caattgggat 840

ttgtcatttt ggatattgtg ggcttttttg gctattcata aagttttttt tatttttttt 900

ttatttaatt ttcagtcaaa tggaaacaag tccaccataa tacacttgtg tttcttttgt 960

caaacttatc aatttgtgtc tgtagatttc aattacaata catattttaa aggccaaaaa 1020

aaaaaaaaaa aaaaaaaa 1038

<210> 3
<211> 710
<212> DNA
<213> Artificial

<220>
<223> alpha subunit sequence

<400> 3

gaagacactc atcacgctcc gccggaagtc gaggacaaag ccatcatgtt ttggacaaga 60

tacgctgaag caagcatttt cttgttgtta atgattcttc atgtcggaca actgtattca 120

agaaacgatg tgtctaacta tggatgtgaa gagtgcaaac tcaagatgaa cgaacgtttc 180

tccaaacccg gggctccggt ctatcagtgc gtgggctgct gcttttcgag agcttacccc 240

acacccctga ggtccaagaa aaccatgctt gtcccaaaaa acatcacatc agaagccact 300

tgctgtgtag caaaagaatc taaaatggtt gccacgaata tcccactata caaccacaca 360

gactgccact gcagcacctg ttactatcat aagtcttaaa acacactctc ttcacatttc 420

tcaaatgctc atttcctgtt cttaaatcac agtgactcat gaaatatgat ttttatgtag 480

ctttccatat ttcaactgtg gccatttcca attcgtttct aaaatggttg gcataagtat 540

tgtaaactgc atattctgtc actatccctt taagagcgta atatgccatc ctttactatc 600

attaaatcgc ttatttattt tgttgccttt actgtgacat tcttcaaatc tataaatgaa 660

ataaaagatt gctgaaggca aaaaaaaaaa aaaaaaaaaa aaaaaaaaaa 710

<210> 4
<211> 27
<212> DNA
<213> Artificial

<220>
<223> Upper-LHbeta/EcoRI primer

<400> 4
caaccgaatt caacgccttc aagatgt 27

<210> 5
<211> 24
<212> DNA
<213> Artificial

<220>
<223> Lower-LHbeta/EcoRV primer

<400> 5
ccgatatcta gtatgcgggg aaat 24

<210> 6
<211> 20
<212> DNA
<213> Artificial

<220>
<223> Upper-FSHbeta3 primer

<400>  6
aggatgcgtc tgcttgttct                                                    20


<210>  7
<211>  25
<212>  DNA
<213>  Artificial

<220>
<223>  Lower-FSHbeta2/3 primer

<400>  7
tgttgttaag gtcatgatac agtgc                                              25


<210>  8
<211>  20
<212>  DNA
<213>  Artificial

<220>
<223>  Upper-alpha1 primer

<400>  8
gtcgaggaca aagccatcat                                                    20


<210>  9
<211>  21
<212>  DNA
<213>  Artificial

<220>
<223>  Lower-alpha1 primer

<400>  9
tgccaaccat tttagaaacg a                                                  21


<210>  10
<211>  20
<212>  DNA
<213>  Artificial

<220>
<223>  Upper-Activin A primer

<400>  10
tgctgcaagc gacaatttta                                                    20


<210>  11
<211>  19
<212>  DNA
<213>  Artificial

<220>
<223>  Lower-Activin A primer

<400>  11
cattcgtttc ggactcaag                                                    19


<210>  12
<211>  20
<212>  DNA
<213>  Artificial

<220>
<223>  Upper-Activin B primer

<400>  12
caacttagat ggacacgctg                                                   20


<210>  13
<211>  20
<212>  DNA
<213>  Artificial

<220>
<223>  Lower-Activin B primer

<400>  13
gtggatgtcg aggtcttgtc                                                   20


<210>  14
<211>  20
<212>  DNA
<213>  Artificial

<220>
<223>  Upper-beta Actin primer

<400>  14
ccccttgttc acaataacct                                                   20


<210>  15
<211>  19
<212>  DNA
<213>  Artificial

<220>
<223>  Lower-beta Actin primer

<400>  15
tctgtggctt tgggattca                                                    19

## Description

The invention relates to the field of fish culture. The invention in particular relates to the field of hormone driven improvement of the development and maturation of eggs and/or sperm in fish.

Many fish species mature in response to environmental factors. These factors such as light cycle, temperature, season, pressure, and energy reserves, are sensed by the animal and control the inhibitory action of hypothalamic centres on the pituitary. In this way the production of gonadotropins by the pituitary is normally depressed and activated only under certain environmental conditions. When activated, the pituitary releases gonadotropins that stimulate the growth and development of both male and female gonads.

For fisheries it is important that maturation can typically be stimulated artificially by regular injections with the same hormones, which consist often of crude pituitary extracts. The regular injections overrule to some extend the environmental triggers for the development of both the male and female gonads. However, the current practice of artificially improving the development and/or maturation of eggs and/or sperm using hormones is not completely satisfactory. For instance, in eels, which are still immature when they commence their spawning migration, the females have to be treated weekly during 3-5 months before oocytes are ripe enough for ovulation. This is a time consuming procedure and stressful for the fish.

The present invention provides a method for improving the development and/or maturation of eggs and/or sperm in fish using hormone administration comprising providing said fish with cells producing said hormone. The cells that are transplanted into the fish release one or more hormones, thereby at least reducing the need for regular injections with the hormone(s) themselves. The cells may be transplanted in various ways as long as the secreted hormone(s) are released into the circulation system and reach all tissues and in particular the sexual organs in sufficient quantity. The cells may be transplanted anywhere in the body. Implants, consisting of hormone(s) producing cells, are typically used to bypass the pituitary gland in order to produce hormones such as luteinizing hormone (LH), follicle stimulating hormone (FSH) or chorionic gonadotropin (CG). The implants with hormone producing cells are preferably inserted into sites that have access to the bloodstream. Preferred methods of insertion are intra-peritoneal, and subcutaneous injection. Sites with access to the bloodstream are very well suited for cells that produce secondary hormones such as LH, FSH, and CG. Transplantation of cells is often used in mammals and a lot of experience has been obtained with respect to methods to transplant and maintain cells for at least some time. Considering that the fish are typically killed after spawning or harvesting of the ripened eggs or sperm, there is no great need for control over the transplanted cells. Important is that the cells remain present in sufficient numbers to allow complete development and maturation of the gonads. Thus the number of cells may not be less than required for development and maturation and the cells may not be (or become) so numerous that they impede the development and maturation of the gonads or the general health of the animal. The level of production of the hormone by the cells is not very critical. The number of injected cells will depend on the total quantity required for the maturation.. The hormone production will be quantified by a bio assay. The hormone release of the injected cells needs to be sufficiently high to stimulate the development and/or maturation of eggs and/or sperm. The upper boundary for expression of the hormone is not critical as over-expression of a fertility hormone is not toxic in itself and does not negatively affect the stimulation of maturation and/or development of eggs and/or sperm. In the mammalian world, several types of grafting aids have been developed to allow for prolonged stay of the cells or to allow differentiation of the cells. These aids can of course also be used in the present invention. Such aids include, but are not limited to, collagen or synthetic matrices for the grafting and attachment of cells.

As the cells, in many cases, need not be present for a very long time, it is possible to transplant fish cells from many different species into a recipient fish. If the evolutionary difference between the transplanted cells and the recipient is large, it is likely that the recipient fish will mount an immune response to the transplanted cells (the graft). However, as the cells often need only be present for a limited amount time, such immune response can typically be tolerated. To increase the robustness and predictability of the procedure it is preferred that the graft is derived from the same genus or family as the recipient fish species. Preferably, the two are from the same species. As fish are typically outbred populations there are immunological differences between fish of the same species. This is typically not a problem, however, it is possible to further match the graft and the recipient for common immunological markers. Typical markers are major and minor histocompatibility antigens. The grafting of the transplanted cells may further be facilitated by providing the recipient fish with immunosuppressants such as cyclosporin.

The transplantation of hormone producing cells of the invention can be used for improving the maturation of eggs and/or sperm, the fertility of the eggs and/or sperm, the insemination of eggs, the quality of the resulting embryos, the survival of fertilized and unfertilised eggs and the survival of embryos. These improvements all lead to improved development and maturation of eggs and/or sperm in fish. The term development and maturation of eggs and/or sperm in fish is therefore not limited to the natural process of spawning but also relates to artificial methods for egg insemination. Thus, it also relates to the harvesting of unfertilised eggs and/or sperm from fish treated with a method of the invention. The unfertilised eggs may also be used for other purposes than the creation of progeny. A non-limiting example thereof is the production of eggs for human consumption such as caviar.

The development and maturation of eggs and/or sperm in fish can be stimulated in various ways. In one embodiment of the present invention the development and maturation of eggs and/or sperm in fish is said to be stimulated when the absolute number or the quality of the eggs, sperm or embryo's resulting from fertilized eggs is increased.

Reproduction is a highly regulated biological process. Different aspects of reproduction are regulated by different hormones. However several hormones can produce more or less similar effects when expressed by a cell that is transplanted into a fish. These hormones include: Growth hormone, Corticoliberin (Adreno Corticotrope hormone), Thyroid stimulating hormone, FSH, LH, Prolactin, CG (Chorionic gonadotropin), MG (Menopause gonadotropin), Somatotropin or a combination thereof. The hormones mentioned above are also known under different names. As the underlying amino acid sequence is the same, the hormones referred to by the synonyms are also within the scope of the invention. For instance, Growth hormone is sometimes also referred to as Somatotropin, somatotropic hormone, hypophysis growth hormone, somatotropic hormone or STH. Corticoliberin is also referred to as releasing corticotropin hormone. Adreno Corticotrope hormone is also referred to as Corticotropin, adrenocorticotropin, adrenotropin, corticotropin, ACTH or adrenocorticotropic hormone. Thyroid stimulating hormone is also referred to as TSH, thyrotropin or thyrotropic hormone. FSH is also referred to as Follicle stimulating hormone, follitropin or gametocinetic hormone. LH is also referred to as Luteinizing hormone, Luteotropin or interstitial cell stimulating hormone (ICSH). Prolactin is also referred to as PRL, lactogenic hormone, mammotropic hormone, galactopoietic hormone or lactotropin. CG (Chorionic gonadotropin) is also referred to as Chorionic gonadotropic hormone, chorionic gonadotropic hormone, choriogonadotropin or chorionic gonadotropin and Menopause gonadotropin (MG) is also referred to as urogonadotropin, menotropin or Menopause gonadotropic hormone.

In a preferred embodiment of the invention said hormone is a hormone directly involved in the development and maturation of eggs. Such fertility hormones are typically produced by the pituitary, or the sexual organs. In a preferred embodiment the fertility hormone comprises luteinizing hormone (LH), follicle stimulating hormone (FSH), or chorionic gonadotropin (CG) or a functional part, derivative and/or analogue of such a hormone. These hormones are very potent stimulators of the development and maturation of eggs and/or sperm in fish. These hormones are very conserved in nature and hardly have a species barrier. For instance, the presence of human fertility hormones in urine can be detected by incubating them with frog eggs. Similarly, human chorionic gonadotropin (hCG) also works on eel and carp and salmon pituitary extracts work on many different fish species such as eel, seabream and trout.

It is possible that the recipient develops an immune response against a heterologous hormone. Although this immune response is typically too late to affect the stimulation of maturation and/or development of eggs and/or sperm, it is preferred that the hormone is a fish hormone or a functional part, derivative and/or analogue thereof. This limits the divergence between the provided and the endogenous hormone, thereby at least in part limiting the development of an immune response against the provided hormone in the recipient. Preferably, the hormone is derived from a species that belongs to the same genus as the recipient. In this way the chance that an immune response is developed is further reduced. In a particularly preferred embodiment a provided hormone is immunologically identical to the equivalent thereof in the recipient. This completely prevents the development of any detrimental immune response against the provided hormone.

The cells can either express the desired hormone without manipulation or can be manipulated to express the desired hormone. When the cells do not express the hormone already or do not express sufficient hormone, they can be provided with the genetic information for expressing the hormone.

In a preferred embodiment the cells are provided with the genetic information to express the hormone. This can be done by providing the cells with expression cassettes comprising coding sequences for the hormones. However, it is also possible to activate the endogenous genes by inserting an active regulatory sequence near the coding sequence(s) for the respective hormones. This can be done for instance through homologous recombination.

The LH and FSH proteins belong to a family of related proteins. Both share the characteristic that they are functional as heterodimers consisting of a common α-subunit and a different β-subunit. In the case of hormones that consist of more than one protein chain it is possible that cells do not express all of the chains needed to generate the hormone. In these cases only expression cassettes are required for the chain(s) that are lacking. Thus, if one or more but not all of the subunits of the hormone are adequately expressed in the cells one only needs to express the remaining subunit(s) in the cell. If none of the subunits are expressed, one has to manipulate the cells such that all of the subunits are expressed at adequate levels. In a preferred embodiment, the cells are provided with expression cassettes for the subunits of the hormone. In a preferred embodiment the cells are provided with expression cassettes for the three protein chains that make up LH and FSH (i.e. for the common α-subunit and the unique β-subunits for each of the hormones), or in separate cell lines the combination of βLH + α and βFSH + α are expressed. Thus in a preferred embodiment said cells are genetically modified to express said hormone(s). Preferably, the cells are provided with one or more genes encoding said hormone(s).

The cells can be primary cells or cell lines that are cultured in vitro for an extended period. In a preferred embodiment, the cells are derived from a clonal population of cells. In this way, the cells can be subjected to detailed quality control prior to use. This also allows for the generation of cell banks that have the same property. Moreover, a clonal population can be subjected to further manipulations. For instance, if one wants to reduce immune responses in the recipient, it is possible to knock out expression of major and/or minor histocompatibility antigens. Thus in a preferred embodiment, the cells have been selected for a reduced immunogenicity in the recipient.

A method of the invention may be used for all types of fish. Preferred fish are eel, seabass, seabream, halibut, salmon, trout, cod, carp, catfish, and sturgeon. However, the invention is particularly suited for diadromous and preferably semelparous fish. These fish take a long time before spawning and typically do not all respond similarly to outside signals. With a method of the invention it is possible to stimulate the development and maturation of the fish at least in part independently of the environmental stimuli. This introduces a large amount of predictability towards the starting point for the fish culture. In diadromous fish it is further possible to synchronize egg development and maturation such that work can be better scheduled in the production process.

The invention further provides an isolated and/or recombinant fish cell that produces a fertility hormone. In a preferred embodiment said cell is genetically modified to express said hormone. The invention further provides a fish cell provided with the capacity to express a fertility hormone. Preferably, the fish cell is provided with a recombinant and/or isolated nucleic acid sequence encoding said fertility hormone. If the hormone consists of one or more subunits, the fish cell is preferably provided with an isolated and/or recombinant nucleic acid sequence encoding at least one subunit of said hormone. Preferably, the fish cell is provided with nucleic acid sequence encoding all subunits of said hormone. Preferably said cell is a cell of a consumer fish. In a preferred embodiment said cell originates from eel, seabass, seabream, halibut, salmon, trout, cod, carp, catfish or a sturgeon.

The invention further provides a fish that comprises a cell according to the invention. Preferably, said fish is a consumer fish, In a preferred embodiment said fish is an eel, seabass, seabream, halibut, salmon, trout, cod, carp, catfish or a sturgeon.

### Examples

### Cloning of LHβ, FSHβ and α zebrafish genes

The genes of zebrafish are already published LHβ (AY424304), FSHβ (AY424303) and α (AY424306), also the genes of eel are reported for LHβ (AB175835) in *Anguilla japonica;* FSHβ (AY169722) in *Anguilla anguilla* and for α (AB175834) in *Anguilla japonica.* In order to clone LHβ, FSHβ and α primers were designed based on the cDNA sequence of each one:

The sequence used for the design of the primers of the LHβ was:

atatataaat ctggacacgc agagacactt acaacagcct gctgagcaac cgcaacgcct
gtcaagatgt tattggctgg aaatggtgtc ttctttctct tctctttgtt tttcctgctg
gcggctgctc agagcttggt ttttccacgc tgtgagctag taaatgagac ggtatcggtg
gaaaaagagg gctgtccaaa atgcctggtg tttcagacca ccatctgcag cggccactgc
gtaacaaggg atcccgttta caagagcccg ttttccaccg tccaccagac agtgtgcatg
taccgggacg tccgctatga gaccattaac ctgcccgact gttccgccgg cgtggacccg
cagatcacat acccggtggc gctgagctgc gactgcagtc tgtgcaccat aaacacttcc
gactgcacca tccagagcct gcagcccgac ttctgcatgt cccagagaga ggatttcccc
gcatactaga cctcgggcaa ctcacgtcaa cctacgcaca tagtcgagct cagcattatt
agccctcctg tatgtttttt ccattaatat atatactttc aagacactag tattcagctt
aaagtgacat ttaaagacta aactaggtta attaggggga aaagtagagt aagtcattgt
ataatagtgg tttgttctgg agacaatcca aaactaatat tgcttaaggg ggctaataaa

attgacctta aaatgaattt aaataattta aaaactgcat ttattctagt cgaaataaaa
gaaataagac tttctttaga agaaaaaaca ttataggaaa tactgcaaaa aaattcctga
atctgttcaa catcattcgg gaaatcaaag gagggctaat aactgtgact tcagctgtac
atcaataaag aggctggttc ttaaattcaa aaaaaaaaaa aaaaaaaaaa aaaaaaaa

The sequence used for the design of the primers of the FSHβ was:

ggtctccacg aaactcccgc agatgaggat gcgtgtgctt gttctggcgc tgctgttgcc
ggtgttaatg agcgcagaat cagaatgcag gtgcagctgt cgactcacca acatctccat
cactgtggag agcgaagaat gtgggagctg cgtcacaatc gacaccacag cctgtgcagg
actatgctgg acaatggatc gagtttaccc tagttccatg gcacagcaca cccagaaggt
ctgtaacttc aagaacttga tgtacaagag ctacgagttt aaaggctgtc ctgcaggggt
tgattcagtc ttcgtgtacc ccgtggctct gagctgtgag tgcaaccagg ttaactcaga
cacaacagac tggggagcta tcagcccgca gaccaccagc tgcagcatac actagagcac
tgtatcatga ccttaacaac atgtacgttg cagaatcaaa ttaagtaagg agtacaatta
gaccatttaa ggatatcaat tatttacaaa acctttagtt tttcatgcat cccacacaca
tggtaatttg gttacttgaa ttaatctgtt gtgttaattc tatagttggt actatggtaa
ctagagtact agagtatcca atgctatact agttttaatt acagttaatt atagaaaagt
atgctacagt atttattaca gtttttctgt tttcaatatt tagtactaca gtatgctagt
gcattcatta acaataagct gtaaatacta taataaatac aggttaatac actttactat
agtatgcttg atcaacacta ttatttaatg tgagttacta tagtactttt caattgggat
ttgtcatttt ggatattgtg ggcttttttg gctattcata aagttttttt tatttttttt
ttatttaatt ttcagtcaaa tggaaacaag tccaccataa tacacttgtg tttcttttgt
caaacttatc aatttgtgtc tgtagatttc aattacaata catattttaa aggccaaaaa
aaaaaaaaaa aaaaaaaa

The sequence used for the design of the primers of the α subunit was:

gaagacactc atcacgctcc gccggaagtc gaggacaaag ccatcatgtt ttggacaaga
tacgctgaag caagcatttt cttgttgtta atgattcttc atgtcggaca actgtattca
agaaacgatg tgtctaacta tggatgtgaa gagtgcaaac tcaagatgaa cgaacgtttc
tccaaacccg gggctccggt ctatcagtgc gtgggctgct gcttttcgag agcttacccc
acacccctga ggtccaagaa aaccatgctt gtcccaaaaa acatcacatc agaagccact
tgctgtgtag caaaagaatc taaaatggtt gccacgaata tcccactata caaccacaca
gactgccact gcagcacctg ttactatcat aagtcttaaa acacactctc ttcacatttc
tcaaatgctc atttcctgtt cttaaatcac agtgactcat gaaatatgat ttttatgtag
ctttccatat ttcaactgtg gccatttcca attcgtttct aaaatggttg gcataagtat
tgtaaactgc atattctgtc actatccctt taagagcgta atatgccatc ctttactatc
attaaatcgc ttatttattt tgttgccttt actgtgacat tcttcaaatc tataaatgaa
ataaaagatt gctgaaggca aaaaaaaaaa aaaaaaaaaa aaaaaaaaaa

In each case the gray part represents the coding sequence, and the predicted amplified region is underlined.

The primers designed were:

| | |
|---|---|
| Upper-LHβ/EcoRI | 5'-CAA CCG AAT TCA ACG CCT TCA AGA TGT-3' |
| | |
| Lower- LHβ/EcoRV | 5'-CCG ATA TCT AGT ATG CGG GGA AAT-3' |
| Upper -FSHβ3 | 5'-AGG ATG CGT GTG CTT GTT CT-3' |
| Lower- FSHβ2/3 | 5'-TGT TGT TAA GGT CAT GAT ACA GTG C-3' |
| Upper-α1 | 5'-GTC GAG GAC AAA GCC ATC AT-3' |
| Lower- α1 | 5'-TGC CAA CCA TTT TAG AAA CGA-3' |

To facilitate further cloning steps the LHβ oligos, include restriction enzyme sites for EcoRI and EcoRV in the upper oligo and in the lower oligo respectively.

Total RNA was isolated from zebrafish heads homogenized in liquid nitrogen and extracted using TRIZOL reagent according to the manufacturer's instructions. Traces of DNA were removed by incubation with DNaseI followed by phenol/chloroform extraction and ethanol precipitation. RT-PCR was performed using the Superscript II one step RT-PCR system with platinum Taq. Reactions were performed with 100ng of total RNA using 25 pmol of the upper and lower primers. Reverse transcription was performed at 50 °C for 30 min. PCR conditions were 40 cycles of denaturation at 94 °C for 20 s, annealing at and 55 °C for LHβ and for FSHβ and 50 °C for a during 30 s and extension at 72 °C for 1 min followed by a final extension step at 72 °C for 10 min. The PCR products were separated by electrophoresis in a 1% gel of agarose and stained with ethidium bromide. The FSHβ PCR product produced a faint band when observed in the agarose gel, in order to optimise we did a PCR using as template this PCR product the reaction was performed with 1/20 of the PCR product reaction using 10µM of the upper an lower primers. PCR conditions were 40 cycles of denaturation at 94 °C for 20 s, annealing was performed in a gradient at from 50 °C to 60 °C for 30 s and extension at 72 °C for 1 min followed by a final extension step at 72 °C for 10 min. A sharp band was then observed. To confirm the identity of the amplified sequences, the PCR products were cloned in pCRII-TOPO vector, digested with restriction enzymes to identify the correct direction and sequenced. The analysis of the sequence revealed that we had cloned LHβ, α and FSHβ subunits of zebrafish. These constructs allow now sub-cloning the genes under a constitutive promoter.

Cloning LH*β*, FSHβ and α under the control of a constitutive promoter (CMV). The p3XFLAG-CMV-9 expression vector is used to establish transient or stable fusion proteins. The vector encodes three adjacent FLAG epitopes upstream from the multicloning region. This results in an increased detection using anti-FLAG antibody. The promoter-regulatory region of the CMV drives transcription of flag fusion constructs. The preprotrypsin leader sequence precedes the FLAG sequence, promoting the secretion of the protein. The amino glycoside phosphotransferase gene (Neo) confers resistance to amino glycosides such as Geneticin (G418), allowing for selection of stable transfectants.
We used this vector because it has the advantages that the synthesized proteins will be secreted driven by the preprotrypsin leader, we can detect the expression of the proteins by western blots with the anti-FLAG antibody and we can make stable cell lines selecting with geneticin.

### Cloning of LHβ

The PCR product was purified and digested with EcoRI and EcoRV as well as the p3XFLAG-CMV-9 expression vector. The DNA fragments were ligated overnight at 4 °C. The ligation mixture was then used to transform chemical competent cells. Enzymatic digestions selected positive clones. Those clones that give a correct pattern of digestion were sequenced. At the moment, the clone sequenced showed an incorrect insertion of LHβ so I had to repeat this cloning step. The predicted amino acid sequence gives a protein of 187 amino acids with a molecular weight of 20593.8

### Cloning of FSHβ

pCRII-TOPO FSHβ was digested with BamHI/ NotI the band that corresponds to FSHβ cDNA was purified and sub-cloned in the p3XFLAG-CMV-9. The positive clones were then digested then with Not/EcoRV and the mug bean nuclease and religated to get FSHβ in frame with the preprotrypsin leader, these construct was designated as CMV-FSHβ. Alternatively, pCRII-TOPO FSH*β* was digested with Xba/BamHI and the resulting band was subcloned in the p3XFLAG-CMV-9. The positive clones were then digested then with EcoRV and religated to get FSHβ in frame with the preprotrypsin leader, this construct was designated as CMV-FSHβ. Enzymatic digestions selected positive clones; those clones that give a correct pattern of digestion were sequenced. The sequencing of the two different colonies, which correspond to the different strategies of cloning, was correct. These constructs were used for the transformation of the ZF4 cell line. The predicted amino acid sequence gives a protein of 174/183 amino acids with a molecular weight of 19062.24/19965.21 respectively to the two different strategies of cloning.

### Cloning of α

pCRII-TOPO α was digested with KpnI/XbaI and the resulting band was subcloned in the p3XFLAG-CMV-9. Enzymatic digestions selected positive clones. Those clones that give a correct pattern of digestion were sequenced. The analysis of the sequence revealed that we have cloned a in the correct orientation and in frame with the flag and the preprotrypsin leader sequence this construct received the name CMV-α. The predicted amino acid sequence gives a protein of 190 amino acids with a molecular weight of 21083.24

The plasmids CMV-FSHβ and CMV-α were purified by alkaline lysis with SDS using the QIAprep spin Miniprep kit. The purified plasmids were linearised with ScaI. The linearised products were separated by electrophoresis in a 1% gel of agarose and stained with ethidium bromide. The linearised plasmid was purified and quantified.

### Transfection of FSHβ in zebrafish fibroblast cell line (ZF4)

The ZF4 (ATCC number: CRL-2050) cells are fibroblast from 1 day-old zebrafish embryos. The frozen aliquot of ZF4 cells was removed from the liquid nitrogen and placed immediately on ice for 10 minutes. The thawed cell suspension is removed from the vial and diluted in 10 ml of complete medium (1:1 mixture of Dubelco's modified Eagle's medium and Ham's F12 containing 1.2g/L of sodium bicarbonate, 2.5mM L-glutamine, 15mM HEPES and 0.5mM sodium pyruvate, 10% of foetal bovine serum and penicillin/streptomycin) at room temperature. The supernatant is discarded by centrifugation at 1200 rpm for 8 min. The cells are resuspended in 8 ml of complete medium and transferred to a tissue flask (T25) and cultured at 28°C. The cells are examined under the inverted microscope to check for cell density. In cultures with a confluence of 80% the medium is removed and washed with 3 ml of PBS to remove cellular debris and serum. Then 0.5 ml of trypsin solution (0.25%) is added and incubated at room temperature until the cells from the monolayer detach from the flask, when a single cell suspension has been obtained 5 ml of complete medium is added to stop trypsinisation. Viable and nonviable cells are counted using a Fuchs-Rosenthal hemocytometer. Cells were seeded in new flasks at a density of 100- 150 cells/mm² (5X105 in each T25 flask). The cells were incubated at 28°C for maximum 4 days before the next passage. Transfection was realized in zebrafish fibroblasts when they were 50-60% confluent using Fugene 6 following manufacturer's instructions. The Fugene6/DNA complex is prepared in a 6:1 ratio of Fugene6: DNA in medium without serum. The culture medium is removed from the cells and replaced with serum free medium. The Fugene6/DNA complex is added drop wisely and mixed. The cells are incubated at 28°C for 5 hours. Then the medium is removed and replaced with complete medium. As a control Zf4 cell were cotransfected with the pEYFP-N1 plasmid and analysed for positive cells under the Leica confocal at 16 and 24 hours after transfection. Different concentrations of DNA were used to establish the optimal concentration for this plasmid giving as a result that 1 µg of DNA in a surface of 21 cm² was the best concentration to obtain a transformation of approximately 30%.

### Detection of the expression of LHβ, FSHβ and α

### Immunohistochemistry

Transfected cells were analysed also by immunohistochemistry to detect the expression of the protein in the cells. The cells were fixated with p-formaldehyde 2% glutaraldehyde 0.1% in PBS during 10 minutes, to eliminate the fixative cells are washed twice with PBS. Then they were permeabilised for 10 minutes with 0.2% of Triton X-100 in PBS. To reduce auto-fluorescence caused by the fixative NaBH4 2 mg/ml in PBS was added during 10 min. Blocking was done with 0.1%BSA-c, 0.02% cold water fish skin gelatin during 30 minutes. Incubation with the anti-FLAG antibody (1:250) was performed overnight at 4°C in 0.1%BSA-c, 0.02% cold water fish skin gelatin. The antibody was removed and washed with 0.1%BSA-c, 0.02% cold water fish skin gelatin twice 10 min each. The secondary antibody (anti-Rabbit Alexa 488) is added in a dilution 1:1000 in 0.1%BSA-c, 0.02% cold-water fish skin gelatin and incubated at room temperature during 60 min. Then is washed three times with 0.1%BSA-c, 0.02% cold water fish skin gelatin and mount in DABCO/Gelvatol. The analysis of the cells in the Leica confocal revealed expression of the protein in vesicles in the transfected cells. This localization is in agreement with the expected site for proteins that are going to be secreted. Western Blot
The different constructs were transfected using Fugene 6 in T25 flasks in duplicate, as control cells were not transfected with DNA, transfected with the empty vector and with the positive control CMV- BAP. The proteins were obtained at the third and fifth day after transfection from the supernatant. The supernatant was concentrated using the amicon columns following the manufacturers instructions. FLAG fusion proteins were immunoprecipitated with Anti-FLAG M2 affinity gel, following manufacturers instructions. Proteins samples were diluted 1:4 with sample buffer boiled and kept at -80°C. For setting the conditions of the western blot only the positive (CMV-BAP) and negative (NO DNA) controls were analysed, samples were loaded in a 12% acrilamide gel, run during 60 min at 50 mAmp. The gels were blotted in nitrocellulose membranes. Membrane was blocked with milk 5% overnight at 4°C and immunodetection was realized against anti FLAG antibody at a 1:250 dilution in milk 5% during one hour at room temperature. Immunodetection was revealed with ECL following manufacturer's instructions. The antibody recognized the expressed BAP protein showing a band of the predicted size. With these experiments we show that transient transfected cells are producing the proteins of interest.

### Making stable cell lines

Cells were transfected in 6 well chambers with linearised and purified DNA using Fugene 6. As a control cells were transfected without DNA. After 3 or 5 day after transfection the complete medium was replaced with complete medium with G418 added. The amount of G418 to kill cells that are not expressing the construct varies from cell line to cell line. For the ZF4 cell line the concentrations suggested by the manufacturer are between 0.8 and 1 mg/ml. Cells were treated with 0.8 and 1 mg/ml in quadruplicates, media was changed daily to wash out the dead cells. This was done during 15 days until the plates that contained the cells that did not have DNA were dead and we could not observe any cell in the plate. Then the transfected cells were incubated with the same concentration of G418 for another 5 days. When a confluent monolayer was obtained, the cells were subcultured into a T25 flask and the concentration of G418 was lowered to 0.5mg/ml in complete media. Stable cell lines will be tested with immunohistochemistry and western blot analysis with anti-FLAG antibody to detect the expression of the protein.

### Bioassay

In order to test if the hormones expressed by the transfected cells are active a simple bioassay is going to be performed. Follicular cells cultures from zebrafish respond to pituitary extracts and/or human chorionic gonadotropin (hCG) by upper or down regulating the expression of different genes. hCG (15IU/ml) increases the expression level of activin βA in a time dependent manner. This effect is evident at 40 min of the treatment and reached a maximal level at 2 h, longer treatment (4 h) causes a diminish of the effect. In contrast activin βB is suppressed in the same conditions. When experiments using different concentrations of hCG are performed a dose dependent response is observed. Goldfish pituitary extract also stimulates expression of activin βA and suppresses activin βB in a dose dependent manner. We plan to use this characteristic of the follicular cells in culture to test if the FSHβ and LHβ are active.

### In vitro follicular cell culture

### Isolation of follicular cells

Young zebrafish were purchased from a pet store and maintained without separation of males and females. Females were anaesthetized with 0.01% tricaine methansulfonate solution for 2 minutes or until they were standing still, and decapitated before dissection. The ovaries were then removed and placed in a 10 mm culture dish with L-15 (Gibco). The follicles from 5 females were carefully separated with the aid of insulin needles. The separated follicles were measured with an ocular micrometer in a dissecting microscope and the healthy viotellogenic follicles around 0.45mm were selected, pooled and cultured in T25 flask for 6 days in M199 medium supplemented with 10% foetal bovine serum at 28°C and 5% CO_{2.} The medium is changed on the third day of the incubation. During the 6-day incubation follicle cells proliferated significantly, increasing the yield of cells for the experiments. Cells are washed and trypsinised at 28°C for 15 min. Thereafter the cells are washed three times with medium M199 through centrifugation at 1000 rpm for 2 min, and then subcultured in 24 plate at a density of 1X105 cells/ml per well for 24 hours in complete M199 before hormone treatment. The amount of cells was not enough for the experiment, so we should start with 20 females to get enough material.

### Hormone treatment

Different concentrations of the supernatant will be used, as a positive control hCG will be used at a 15IU/ml, and carp pituitary extracts will also be included. With the pituitary extract condition we can compare the amount of cells that should be used to observe an effect in the reproduction capacity of the female eel.

### In vivo injection

hCG was dissolved in 0.9% of NaCl solution in a concentration of 20IU/ml. Each fish will receive 50µl of saline as a negative control, hCG as a positive control and different concentrations of the supernatant or the purified FSHβ and/or LH*β*. At 1, 2, 4, 6 and 12 h after injections fish are killed and ovaries removed for RNA extraction

### RNA extraction

At the end of the hormonal treatment total RNA was isolated from zebrafish ovaries or follicular cells, homogenized in liquid nitrogen and extracted using TRIZOL reagent according to the manufacturer's instructions. Traces of DNA were removed by incubation with DNaseI followed by phenol/chloroform extraction and ethanol precipitation. RT-PCR was performed using the Superscript II one step RT-PCR system with platinum Taq. Reactions were performed with 100ng of total RNA using 25 pmol of the upper and lower primers. Reverse transcription was performed at 50 °C for 30 min. PCR conditions were 40 cycles of denaturation at 94 °C for 20 s, annealing at 56 °C during 30 s and extension at 72 °C for 1 min followed by a final extension step at 72 °C for 10 min. The PCR products were separated by electrophoresis in a 1% gel of agarose and stained with ethidium bromide.

Designed primers to amplify Activin βA, Activin βB and βActin:

| | |
|---|---|
| Upper-Activin A | 5'-TGC TGC AAG CGA CAA TTT TA -3' |
| Lower-Activin A | 5'-CAT TCG TTT CGG ACT CAA G -3' |
| Upper-Activin B | 5'- CAA CTT AGA TGG ACA CGC TG-3' |
| Lower-Activin B | 5'- GTG GAT GTC GAG GTC TTG TC-3' |
| Upper- β Actin | 5'- CCC CTT GTT CAC AAT AAC CT-3' |
| Lower- β Actin | 5'-TCT GTG GCT TTG GGA TTC A-3' |

### Transplant of the stable cell lines in the eel

Stable cell lines will be transplanted to the eel intraperitoneally. Eels will be anaesthetised and a small incision will be made in the belly. The correct amount of cells that produce a constant amount of hormone will be transplanted. As a negative control cells that do not express hormones and only have the empty vector will be transplanted, and as positive control a group of eels will be injected with pituitary extracts.

### Measurement of the maturation process and egg ripening.

To quantify final maturation of female eels there are different characteristics that change along the maturation process, those are a significant increase of the body weight, increase in the eye diameter and morphological changes during the development of the oocytes. Seven morphological stages of final oocyte maturation are observed during pituitary extract treatments. Non-transparent oocytes (stage 0) are still small and fully filled with fat droplets. Final hydration onsets development into stage 1, showing oocytes with increasing transparency and a centred nucleus. In stage 2, the oocytes are fully transparent and fat droplets are clustering and centering. In stage 3, germinal vesicle (GV) migration occurs. In stage 4, the germinal vesicle is found in the periphery and the fat droplets are located on the opposite side. In stage 5, the fat droplets decrease due to the fat fusion, giving as a result larger fat droplets. At stage 6 meiosis II is completed with disappearance of the germinal vesicle and small numbers of large fat droplets are observed. In stage 7 the oocytes have a single fat droplet. There is a change in the diameter of the oocytes during the first two stages; this is because of the process of hydratation. These morphological changes in the oocyte maturation will allow us to determine the effect of the cell transplantation. After transplantation of the cells, eels will be weighted, and the eye diameter will be measured regularly. During the treatment oocyte samples will be taken in order to determine if the oocytes are responding to the treatment.

### Brief description of the drawings

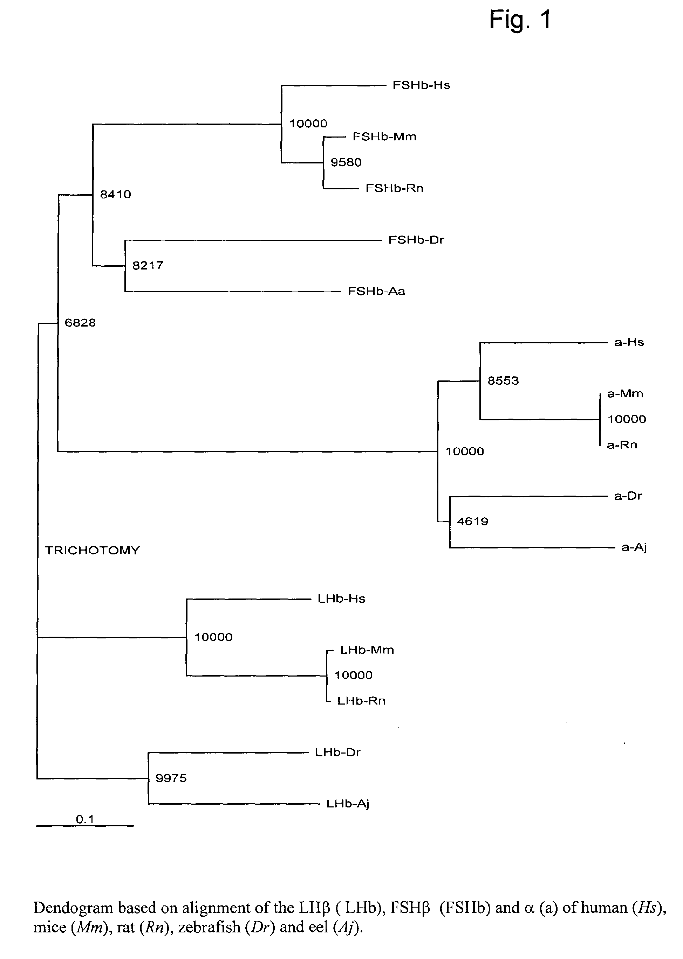

Figure1. Dendrogram based on alignment of the LHβ (LHβ), FSHβ (FSHβ) originating from a (a) of human (Hs), mice (Mm), rat (Rn), zebrafish (Dr) and eel (Aj).

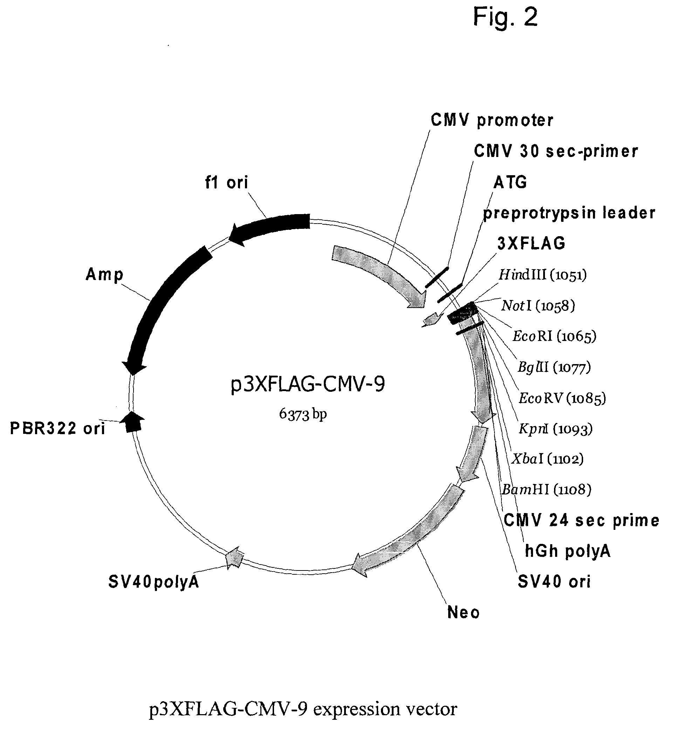

Figure 2. p3XFLAG-CMV-9 expression vector

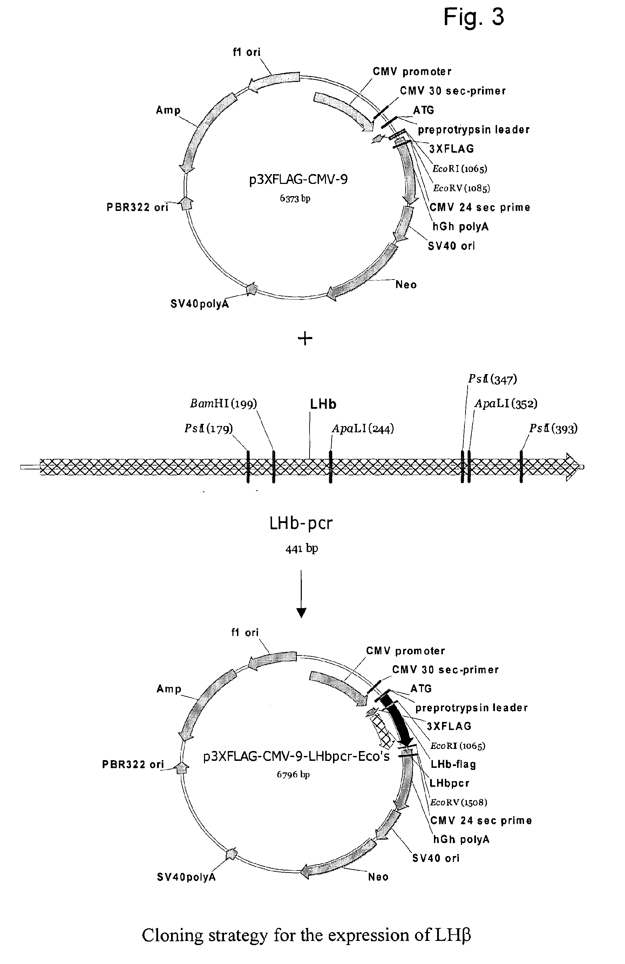

Figure 3. Cloning strategy for the expression of LHβ

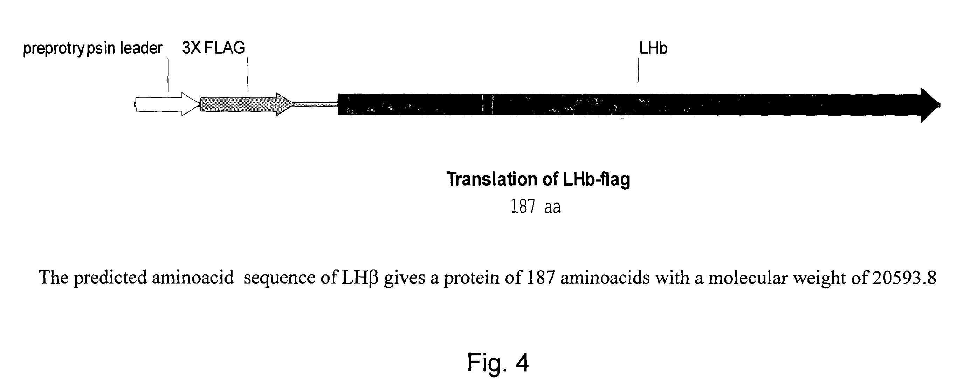

Figure 4. The predicted aminoacid sequence of LHb gives a protein of 187 aminoacids with a molecular weight of 20593.8

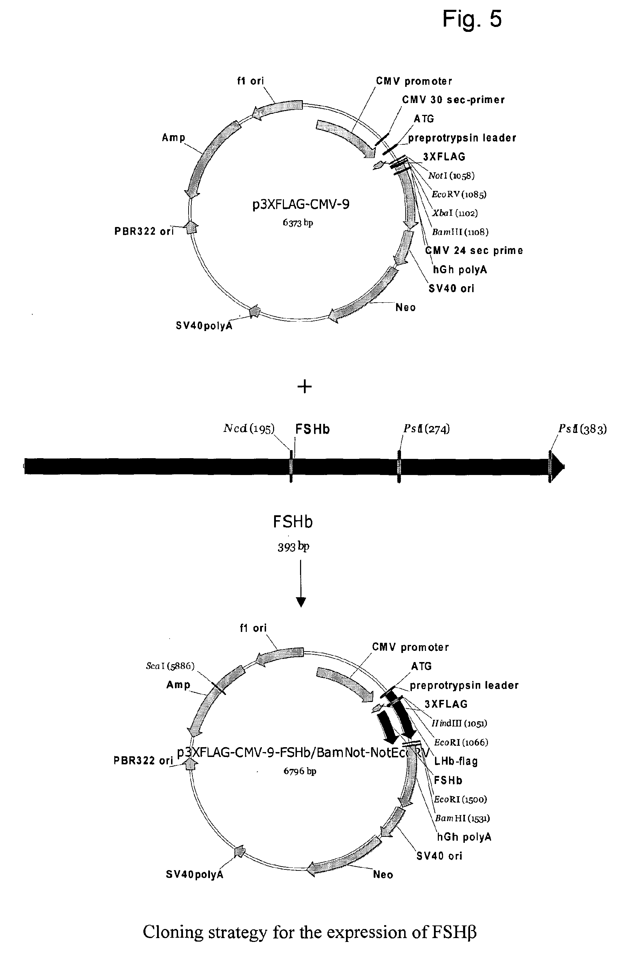

Figure 5. Cloning strategy for the expression of FSHβ

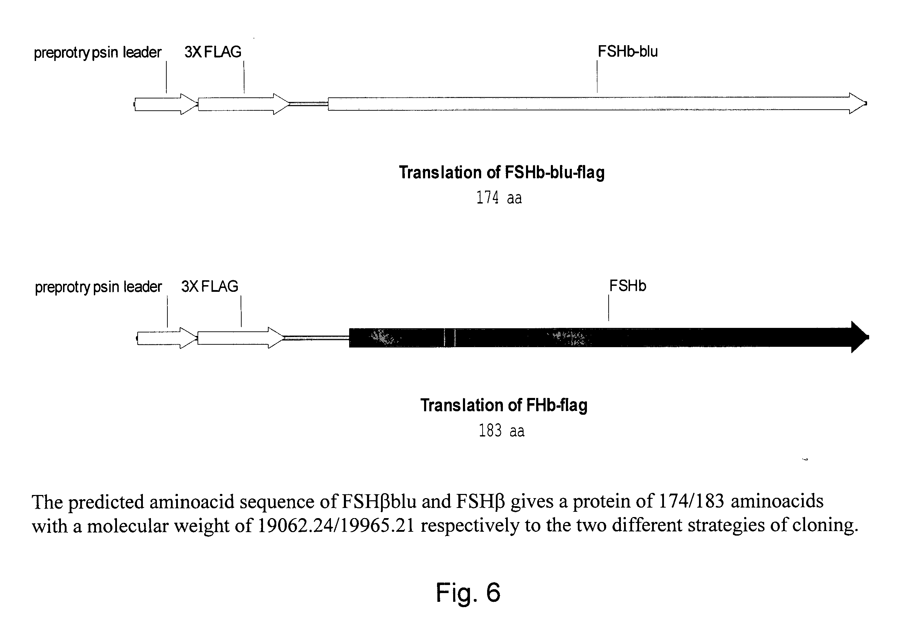

Figure 6. The predicted aminoacid sequence of FSHbblu and FSHb gives a protein of 174/183 aminoacids with a molecular weight of 19062.24/19965.21 respectively to the two different strategies of cloning.

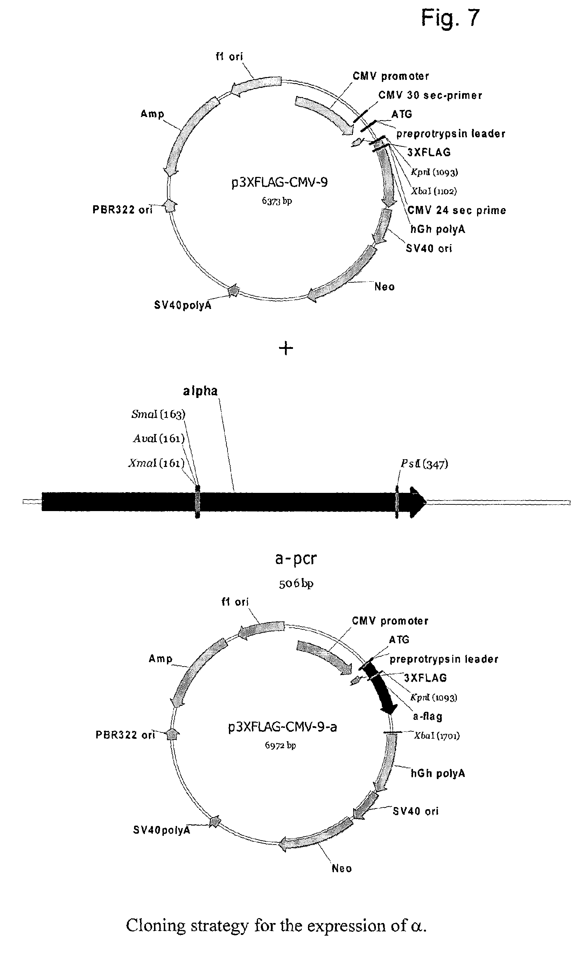

Figure 7. Cloning strategy for the expression of α.

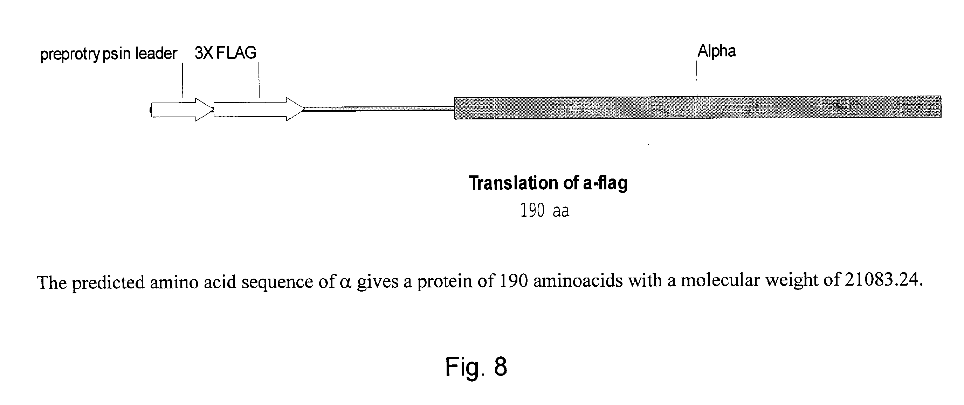

Figure 8. The predicted amino acid sequence gives a protein of 190 amino acids with a molecular weight of 21083.24.

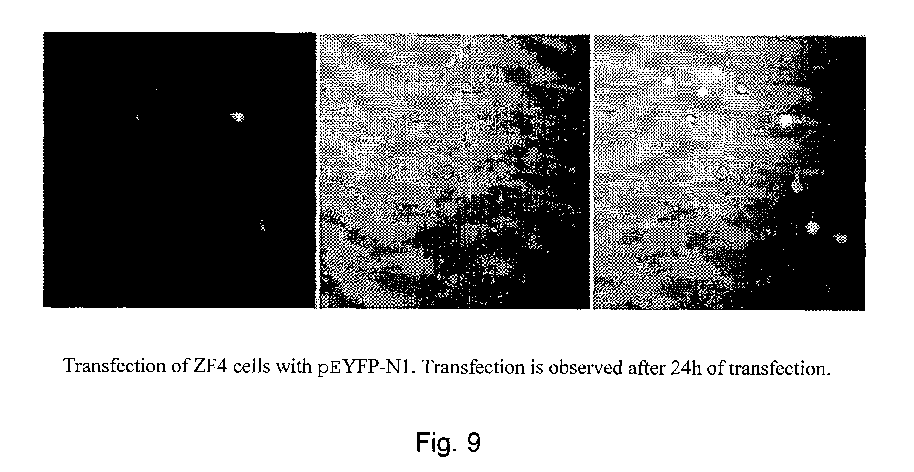

Figure 9. Transfection of ZF4 cells with pEYFP-N1. Transfection is observed after 24h of transfection.

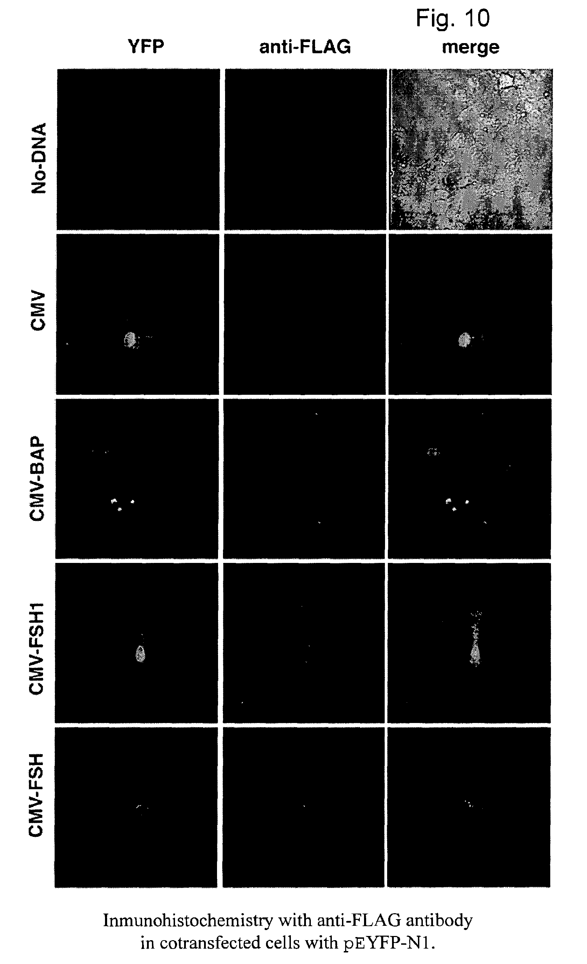

Figure 10. Immunohistochemistry with anti-FLAG antibody in cotransfected cells with pEYFP-N1.

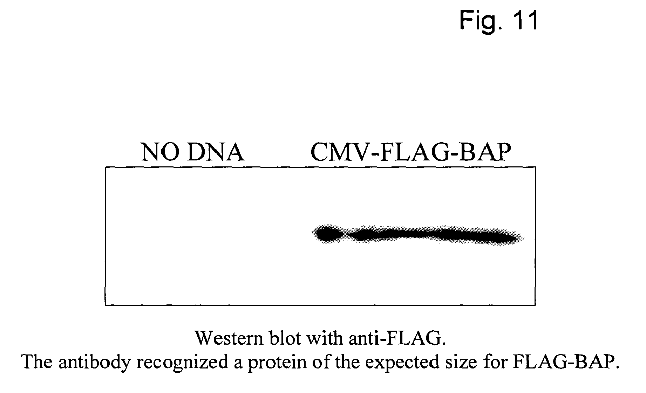

Figure 11. Western blot with anti-FLAG. The antibody recognized a protein of the expected size for FLAG-BAP.

## Claims

1. A method for improving the development and/or maturation of eggs and/or sperm in fish using hormone administration comprising providing said fish with cells producing said hormone.

2. A method according to claim 1, wherein said hormone comprises luteinizing hormone (LH), follicle stimulating hormone (FSH)or chorionic gonadotropin (CG) or a functional part, derivative and/or analogue thereof.

3. A method according to claim 1 or claim 2, wherein said cells are genetically modified to express said hormone.

4. A method according to any one of claims 1-3, wherein said hormone is derived from the same genus as said fish.

5. A method according to any one of claims 1-4, wherein said cells have been provided with one or more genes encoding said hormone.

6. A method according to any one of claims 1-5, wherein said cells are a clonal population.

7. A method according to any one of claims 1-6, wherein said cells have been selected for a reduced immunogenicity in said fish.

8. A method according to any one of claims 1-7, wherein said fish are diadromous fish.

9. An isolated and/or recombinant fish cell that produces a fertility hormone.

10. A fish cell according to claim 9, wherein said cell is genetically modified to express said hormone.

11. A fish cell according to claim 9 or claim 10, wherein said cell is an eel cell.

12. A fish that comprises a cell according to any one of claims 9-11.

13. A fish according to claim 12, that is a consumer fish.

14. A fish according to claim 13 that is an eel.

15. A fish according to claim 14, wherein said eel belongs to the genus Anguilla.
